# EUROPEAN PATENT APPLICATION

(11) **EP 4 697 341 A1**
(43) Date of publication of application: **18.02.2026**
(21) Application number: 24195053.4
(22) Date of filing: 16.08.2024
(51) Int. Cl.: G16H 20/17, H04L 9/40, A61M 5/14

(54) **HEALTH MONITORING SYSTEM, METHOD AND APPARATUS**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application discloses a health monitoring system, a method and an apparatus. The control device 11 connects to the first health monitoring device 12 and the first drug infusion device 13 respectively for mutual authentication, and activates the first health monitoring device 12 and the first drug infusion device 13. The control device 11 sends the first identification information of the first health monitoring device 12 to the first drug infusion device 13. The first drug infusion device 13 establishes a connection to the first health monitoring device 12 based on the first identification information, and the first health monitoring device 12 obtains the target health monitoring data of the first user and sends the target health monitoring data to the first drug infusion device 13. The first drug infusion device 13 sends the target health monitoring data to the control device 11, and infuses the drug to the first user based on the drug infusion index from the control device 11. As a result, the intelligence and convenience of the health monitoring system are improved. The problem of poor reliability in health monitoring caused by the inability of patients to use the control device 11 is finally avoided.

## Description

### TECHNICAL FIELD

The present application relates to the technical field of health monitoring, in particular to a health monitoring system, a method and an apparatus.

### BACKGROUND

Nowadays, there are more and more people suffering from chronic diseases, and people suffering from chronic diseases generally need to take or inject drugs for a long time to relieve their conditions. In the chronic disease treatment market, the health monitoring device and the drug infusion device have already gained some popularity. The health monitoring device is used to obtain health monitoring data, and the drug infusion device is used to infuse the drug into the human body. The health monitoring device may be an analyte monitoring device, such as blood glucose monitoring device, analyte sensor, glucose detection sensor, and general inspection and monitoring device. Generally, the inspection and monitoring device may be a height and weight meter, sphygmomanometer, blood glucose meter, and oximeter. The first drug infusion device may be an infusion delivery device, e.g., insulin infusion pump (POD).

In the prior art, the health monitoring device and the drug infusion device are used as independent devices, and each is matched with a corresponding supporting terminal. The health monitoring terminal is used to control the health monitoring device to perform the corresponding functions and display the health monitoring data transmitted by the health monitoring device. The drug infusion terminal is used to control the drug infusion device to infuse the drug into the human body. When the patient finds an abnormality in his or her health monitoring data when using the health monitoring terminal, the drug infusion is controlled through the drug infusion handheld terminal.

The problem in the prior art is that the patient needs to control multiple terminals, for example, controlling health monitoring terminal to implement health monitoring, and implementing drug infusion by controlling drug infusion terminal via manual control. The overall health monitoring system is poorly intelligent and convenient.

### SUMMARY

The present application provides a health monitoring system, a method and an apparatus to solve the problem of poor intelligence and convenience of existing health monitoring solutions.

According to a first aspect, the present application provides a health monitoring system, where the system includes a control device, a first health monitoring device, and a first drug infusion device;
the control device is configured to connect to the first health monitoring device and the first drug infusion device respectively for mutual authentication, and activate the first health monitoring device and the first drug infusion device;
the first health monitoring device is configured to broadcast a first data packet, where the first data packet contains first identification information of the first health monitoring device;
the control device is configured to receive the first data packet, obtain the contained first identification information, and send the first identification information to the first drug infusion device;
the first drug infusion device is configured to scan a connectable health monitoring device, match with the first health monitoring device based on the first identification information, and establish a connection to the first health monitoring device;
the first health monitoring device is configured to obtain target health monitoring data of a first user and send the target health monitoring data to the first drug infusion device;
the first drug infusion device is configured to send the target health monitoring data to the control device, receive a drug infusion index corresponding to the target health monitoring data from the control device, and infuse the drug to the first user based on the drug infusion index.

In the present application, the user wearing the first health monitoring device is referred to as the first user, and the health monitoring data of the first user obtained by the first health monitoring device is referred to as the target health monitoring data.

According to a second aspect, the present application provides a health monitoring method, where the method is applied to a first drug infusion device and includes:
scanning, by the first drug infusion device, a connectable health monitoring device, matching with a first health monitoring device based on first identification information of the first health monitoring device, and establishing a connection to the first health monitoring device, where a control device receives a first data packet broadcast by the first health monitoring device, obtains the contained first identification information, and sends the first identification information to the first drug infusion device;
receiving target health monitoring data of a first user from the first health monitoring device, sending the target health monitoring data to the control device, receiving a drug infusion index corresponding to the target health monitoring data from the control device, and infusing the drug to the first user based on the drug infusion index.

According to a third aspect, the present application provides a health monitoring method, where the method is applied to a first health monitoring device and includes:
broadcasting, by the first health monitoring device, a first data packet, where the first data packet contains first identification information of the first health monitoring device; a control device receives the first data packet, obtains the contained first identification information, and sends the first identification information to a first drug infusion device; the first drug infusion device scans a connectable health monitoring device, matches with the first health monitoring device based on the first identification information, and establishes a connection to the first health monitoring device;
obtaining target health monitoring data of a first user and sending the target health monitoring data to the first drug infusion device; the first drug infusion device sends the target health monitoring data to the control device, receives a drug infusion index corresponding to the target health monitoring data from the control device, and infuses the drug to the first user based on the drug infusion index.

According to a fourth aspect, the present application provides a health monitoring method, where the method is applied to a control device and includes:
connecting, by the control device, to the first health monitoring device and the first drug infusion device respectively for mutual authentication, and activating the first health monitoring device and the first drug infusion device;
receiving a first data packet broadcast by the first health monitoring device, obtaining the contained first identification information, and sending the first identification information to the first drug infusion device; the first drug infusion device scans a connectable health monitoring device, matches with the first health monitoring device based on the first identification information, and establishes a connection to the first health monitoring device;
receiving target health monitoring data of a first user from the first drug infusion device, where the target health monitoring data is sent from the first health monitoring device to the first drug infusion device;
determining a drug infusion index corresponding to the target health monitoring data, and sending the drug infusion index to the first drug infusion device, so that the first drug infusion device infuses the drug to the first user based on the drug infusion index.

According to a fifth aspect, the present application provides a health monitoring apparatus, where the apparatus is applied to a first drug infusion device and includes:
a scanning unit, configured to scan a connectable health monitoring device, match with a first health monitoring device based on first identification information of the first health monitoring device, and establish a connection to the first health monitoring device, where a control device receives a first data packet broadcast by the first health monitoring device, obtains the contained first identification information, and sends the first identification information to the first drug infusion device;
a drug infusion unit, configured to receive target health monitoring data of a first user from the first health monitoring device, send the target health monitoring data to the control device, receive a drug infusion index corresponding to the target health monitoring data from the control device, and infuse the drug to the first user based on the drug infusion index.

According to a sixth aspect, the present application provides a health monitoring apparatus, where the apparatus is applied to a first health monitoring device and includes:
a broadcasting unit, configured to broadcast a first data packet, where the first data packet contains first identification information of the first health monitoring device; a control device receives the first data packet, obtains the contained first identification information, and sends the first identification information to a first drug infusion device; the first drug infusion device scans a connectable health monitoring device, matches with the first health monitoring device based on the first identification information, and establishes a connection to the first health monitoring device;
an obtaining unit, configured to obtain target health monitoring data of a first user and send the target health monitoring data to the first drug infusion device; the first drug infusion device sends the target health monitoring data to the control device, receives a drug infusion index corresponding to the target health monitoring data from the control device, and infuses the drug to the first user based on the drug infusion index.

According to a seventh aspect, the present application provides a health monitoring apparatus, where the apparatus is applied to a control device and includes:
an activation unit, configured to connect to the first health monitoring device and the first drug infusion device respectively for mutual authentication, and activate the first health monitoring device and the first drug infusion device;
a first receiving unit, configured to receive a first data packet broadcast by the first health monitoring device, obtain the contained first identification information, and send the first identification information to the first drug infusion device; the first drug infusion device scans a connectable health monitoring device, matches with the first health monitoring device based on the first identification information, and establishes a connection to the first health monitoring device;
a second receiving unit, configured to receive target health monitoring data of a first user from the first drug infusion device, where the target health monitoring data is sent from the first health monitoring device to the first drug infusion device;
a sending unit, configured to determine a drug infusion index corresponding to the target health monitoring data, and send the drug infusion index to the first drug infusion device, so that the first drug infusion device infuses the drug to the first user based on the drug infusion index.

According to an eighth aspect, the present application provides a first drug infusion device, where it includes a processor, a communication interface, a memory, and a communication bus, and the processor, the communication interface and the memory communicate with each other through the communication bus;
the memory is configured to store the computer program;
the processor is configured to implement the steps of the method when executing the program stored in the memory.

According to a ninth aspect, the present application provides a health monitoring device, where it includes a processor, a communication interface, a memory, and a communication bus, and the processor, the communication interface and the memory communicate with each other through the communication bus;
the memory is configured to store the computer program;
the processor is configured to implement the steps of the method when executing the program stored in the memory.

According to a tenth aspect, the present application provides a control device, where it includes a processor, a communication interface, a memory, and a communication bus, and the processor, the communication interface and the memory communicate with each other through the communication bus;
the memory is configured to store the computer program;
the processor is configured to implement the steps of the method when executing the program stored in the memory.

According to an eleventh aspect, the present application provides a computer-readable storage medium, where a computer program is stored on the computer-readable storage medium, and the computer program is executed by a processor to implement the steps of the method.

The above technical solutions have the following advantages or beneficial effects:
In the present application, the health monitoring system includes a control device, a first health monitoring device, and a first drug infusion device. The control device connects to the first health monitoring device and the first drug infusion device respectively for mutual authentication, and activates the first health monitoring device and the first drug infusion device. The control device obtains the first identification information of the first health monitoring device based on the first data packet broadcast by the first health monitoring device, and sends the first identification information to the first drug infusion device. The first drug infusion device establishes a connection to the first health monitoring device based on the first identification information. The control device determines a corresponding drug infusion index based on the target health monitoring data of the first user obtained by the first health monitoring device, and controls the first drug infusion device to infuse the drug to the first user based on the drug infusion index. Compared with the solution in which the patient uses multiple handheld terminals to realize health monitoring and drug infusion by manual control, the intelligence and convenience of the health monitoring system are improved. The problem of poor reliability in health monitoring caused by the inability of patients to use the control device is finally avoided.

### BRIEF DESCRIPTION OF DRAWINGS

To illustrate the technical solutions in embodiments of the present application more clearly, the drawings to be used for describing the embodiments are introduced briefly in the following. Apparently, the drawings in the following description are only some embodiments of the present application, and persons of ordinary skill in the art can derive other drawings from these drawings without creative efforts.
FIG. 1 is a structural diagram of a health monitoring system according to the present application;
FIG. 2 is a structural diagram of a health monitoring system according to the present application;
FIG. 3 is a structural diagram of a health monitoring system according to the present application;
FIG. 4 is a structural diagram of a health monitoring system according to the present application;
FIG. 5 is a schematic diagram of a health monitoring process according to the present application;
FIG. 6 is a schematic diagram of a health monitoring process according to the present application;
FIG. 7 is a schematic diagram of a health monitoring process according to the present application;
FIG. 8 is a structural diagram of a health monitoring apparatus according to the present application;
FIG. 9 is a structural diagram of a health monitoring apparatus according to the present application;
FIG. 10 is a structural diagram of a health monitoring apparatus according to the present application;
FIG. 11 is a flow chart of first activation and use of a drug infusion device and a health monitoring device according to the present application;
FIG. 12 is a flow chart of replacement of a health monitoring device according to the present application;
FIG. 13 is a flow chart of replacement of a drug infusion device according to the present application;
FIG. 14 is a flow chart of reconnection of a control device according to the present application;
FIG. 15 is a structural diagram of a first drug infusion device according to the present application;
FIG. 16 is a structural diagram of a first health monitoring device according to the present application;
FIG. 17 is a structural diagram of a control device according to the present application.

### DESCRIPTION OF EMBODIMENTS

To make the objectives and embodiments of the present application clearer, the exemplary embodiments of the present application will be clearly and completely described below in combination with the drawings in the exemplary embodiments of the present application. Obviously, the exemplary embodiments described are only some rather than all of the embodiments of the present application.

It should be noted that the brief description of terms in the present application is only for the convenience of understanding the embodiments described next, and does not intend to limit the embodiments of the present application. Unless otherwise stated, these terms shall be construed according to their ordinary and usual meanings.

Unless otherwise indicated, the terms "first", "second" and "third" in the specification, claims and drawings of the present application are used to distinguish similar or homogeneous objects or entities, and do not necessarily mean to limit a specific sequence or precedence. It should be understood that the terms so used are interchangeable where appropriate.

The terms "include" and "have" and any variations thereof are intended to cover but not exclusively include, for example, a product or device that includes a range of components need not be limited to all components expressly listed, but may include other components not expressly listed or inherent to the product or device.

The term "module" refers to any known or later developed hardware, software, firmware, artificial intelligence, fuzzy logic, or combination of hardware or/and software code capable of performing the functions associated with the component.

Finally, it should be noted that the above embodiments are only used to illustrate the technical solutions of the present application and not to limit them. Although the present application is described in detail with reference to the above embodiments, those skilled in the art shall understand that they can still modify the technical solutions recorded in the above embodiments or equivalently replace part or all of the technical features therein. These modifications or replacements do not depart from the scope of the technical solutions in embodiments of the present application in essence.

For ease of explanation, the above description is made in connection with specific embodiments. However, the above exemplary discussion is not intended to be exhaustive or to limit embodiments to the specific forms disclosed above. Various modifications and variations may be obtained according to the above teachings. The above embodiments are selected and described to better explain the principles and practical applications, so that those skilled in the art can make better use of the embodiments and various variants suitable for specific use considerations.

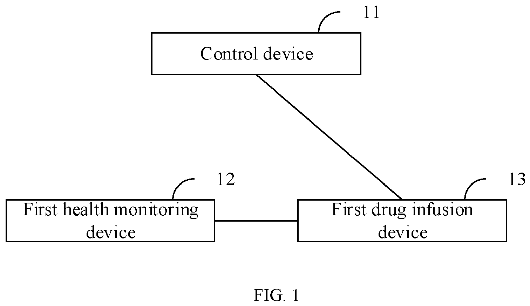

FIG. 1 is a structural diagram of a health monitoring system according to the present application, where the system includes a control device 11, a first health monitoring device 12, and a first drug infusion device 13;
the control device 11 is configured to connect to the first health monitoring device 12 and the first drug infusion device 13 respectively for mutual authentication, and activate the first health monitoring device 12 and the first drug infusion device 13;
the first health monitoring device 12 is configured to broadcast a first data packet, where the first data packet contains first identification information of the first health monitoring device;
the control device 11 is configured to receive the first data packet, obtain the contained first identification information, and send the first identification information to the first drug infusion device 13;
the first drug infusion device 13 is configured to scan a connectable health monitoring device, match with the first health monitoring device 12 based on the first identification information, and establish a connection to the first health monitoring device 12;
the first health monitoring device 12 is configured to obtain target health monitoring data of a first user and send the target health monitoring data to the first drug infusion device 13;
the first drug infusion device 13 is configured to send the target health monitoring data to the control device 11, receive a drug infusion index corresponding to the target health monitoring data from the control device 11, and infuse the drug to the first user based on the drug infusion index.

In the present application, the control device 11 may be a mobile terminal device such as mobile phone, tablet computer and personal digital assistant (PDA, a portable electronic device), or an independent physical server 14, or a cloud server 14 for basic cloud computing services such as cloud database and cloud storage. The control device 11 connects to the first drug infusion device 13 in a wired or wireless way.

The control device 11 is configured to connect to the first health monitoring device 12 and the first drug infusion device 13 respectively for mutual authentication, and activate the first health monitoring device 12 and the first drug infusion device 13. The control device 11 may activate the first health monitoring device 12 and the first drug infusion device 13 through the near field communication (NFC) technology. The first health monitoring device 12 broadcasts a first data packet, and the first data packet contains first identification information of the first health monitoring device 12. The first identification information may be the media access control address (MAC address, a physical address) information, device serial number information, name information, and model information of the first health monitoring device 12. The control device 11 receives the first data packet, obtains the contained first identification information, and sends the first identification information to the first drug infusion device 13. The first drug infusion device 13 scans a connectable health monitoring device, matches with the first health monitoring device 12 based on the first identification information, and establishes a connection to the first health monitoring device 12. The first health monitoring device obtains the target health monitoring data of the first user and sends the target health monitoring data to the first drug infusion device 13. The first drug infusion device 13 sends the target health monitoring data to the control device 11. The control device 11 determines a corresponding drug infusion index based on the target health monitoring data, where the drug infusion index includes drug infusion rate and drug infusion amount. The control device 11 then sends the drug infusion index to the first drug infusion device 13, and the first drug infusion device 13 infuses the drug to the first user based on the drug infusion index. The first health monitoring device 12 and the first drug infusion device 13 may be connected wirelessly, for example, Bluetooth.

The function of the first health monitoring device 12 is to obtain target health monitoring data of a first user and send the target health monitoring data to the first drug infusion device 13. Taking the first health monitoring device 12 as a blood glucose monitoring device as an example, the obtained target health monitoring data of the first user may be blood glucose level data (mmol/L). The first health monitoring device 12 sends the obtained blood glucose level data (mmol/L) of the first user to the first drug infusion device 13.

The first drug infusion device 13 may be various infusion device applicable to drug infusion, for example, an insulin infusion pump (POD). The function of the first drug infusion device 13 is to receive target health monitoring data from the first health monitoring device 12 and send the target health monitoring data to the control device 11. The function of the control device 11 is to determine a corresponding drug infusion index based on the target health monitoring data and send the drug infusion index to the first drug infusion device 13, so that the first drug infusion device 13 infuses the drug to the first user based on the drug infusion index. It should be noted that when the target health monitoring data received by the control device 11 changes, the determined corresponding drug infusion index is adjusted accordingly.

The devices and systems in the health monitoring application scenario are described below.

The health monitoring system may include continuous glucose monitoring (CGM) system, POD, and terminal device. The continuous glucose monitoring system is used to continuously and dynamically detect the changes in blood glucose. It includes blood glucose monitoring device and personal digital assistant (PDA). The blood glucose monitoring device may comprise a glucose detection sensor, an electronic component (mainly including antenna circuit and battery), and an insertion device. The glucose detection sensor can be inserted below the surface of human skin by the insertion device to sense the glucose level. The electronic component is connected to the sensor and can transmit the signal sensed by the sensor to the PDA for displaying changes in blood glucose or to drug infusion device. The insertion device is used to insert the sensor into body fluid below the surface of human skin to detect the analyte level of the user.

The drug infusion device may be insulin injection pump used to infuse insulin, and the insulin pump pumps the liquid drug (for example, insulin) into the patient's body through a needle (for example, a soft needle) with a hollow structure. The needle is inserted into the subcutaneous tissue for insulin delivery. The closed-loop system composed of the continuous glucose monitoring system, drug infusion device and PDA can realize automatic monitoring of blood glucose level and automatic infusion of insulin, to provide a convenient, safe and quick physical health monitoring method for diabetic patients.

In the present application, the health monitoring system includes a control device 11, a first health monitoring device 12, and a first drug infusion device 13. The control device 11 connects to the first health monitoring device 12 and the first drug infusion device 13 respectively for mutual authentication, and activates the first health monitoring device 12 and the first drug infusion device 13. The control device 11 obtains the first identification information of the first health monitoring device 12 based on the first data packet broadcast by the first health monitoring device 12, and sends the first identification information to the first drug infusion device 13. The first drug infusion device 13 establishes a connection to the first health monitoring device 12 based on the first identification information. The control device 11 determines a corresponding drug infusion index based on the target health monitoring data of the first user obtained by the first health monitoring device 12, and controls the first drug infusion device 13 to infuse the drug to the first user based on the drug infusion index. Compared with the solution in which the patient uses multiple handheld terminals to realize health monitoring and drug infusion by manual control, the intelligence and convenience of the health monitoring system are improved.

The present application discloses a health monitoring system, a method and an apparatus. The control device 11 connects to the first health monitoring device 12 and the first drug infusion device 13 respectively for mutual authentication, and activates the first health monitoring device 12 and the first drug infusion device 13. The control device 11 generates a set of random numbers as authentication information, encrypts them, and sends them to the first health monitoring device 12 and the first drug infusion device 13. The control device 11 sends the first identification information of the first health monitoring device 12 to the first drug infusion device 13. The first drug infusion device 13 connects to the first health monitoring device 12 for mutual authentication based on the first identification information and the authentication information, to realize secure transmission of user monitoring data. The present application further discloses the intelligent cooperation capability among a control device, a health monitoring device and a drug infusion device, including the interaction process among the three devices in the device removal and replacement. Compared with the solution in which the patient uses multiple handheld terminals to realize health monitoring and drug infusion by manual control, the intelligence and convenience of the health monitoring system are improved. The problem of poor reliability in health monitoring caused by the inability of patients to use the control device is finally avoided.

In the present application, the first health monitoring device 12 is configured to obtain target health monitoring data of a first user, encrypt the target health monitoring data with a first shared key, and send the encrypted target health monitoring data to the first drug infusion device 13;
the first drug infusion device 13 is configured to decrypt the target health monitoring data with the first shared key, encrypt the decrypted data with a second shared key, and send the data encrypted with the second shared key to the control device 11, so that the control device decrypts the data with the second shared key after receiving the data to obtain the target health monitoring data;
the first drug infusion device 13 receives a drug infusion index corresponding to the target health monitoring data encrypted with a third shared key from the control device 11, decrypts the data with the third shared key to obtain the drug infusion index, and infuses the drug to the first user based on the drug infusion index.

The first health monitoring device 12 obtains target health monitoring data of a first user, encrypts the target health monitoring data with the first shared key, and sends the encrypted target health monitoring data to the first drug infusion device 13. The first drug infusion device 13 decrypts the received data with the first shared key to obtain the target health monitoring data, encrypts the obtained target health monitoring data with a second shared key, and sends the encrypted target health monitoring data to the control device 11. The control device 11 decrypts the receives data with the second shared key to obtain the target health monitoring data. The control device 11 determines a drug infusion index corresponding to the target health monitoring data, encrypts the drug infusion index with a third shared key, and sends the encrypted drug infusion index to the first drug infusion device 13. The first drug infusion device 13 decrypts the data with the third shared key to obtain the drug infusion index, and infuses the drug to the first user based on the drug infusion index.

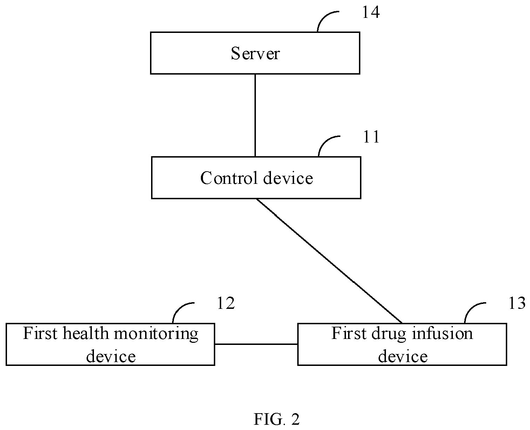

FIG. 2 is a structural diagram of a health monitoring system according to the present application, where the system further includes a server 14, and a control device 11 is connected to the server 14;
the control device 11 is configured to awaken the first drug infusion device 13 through NFC;
the first drug infusion device 13 is configured to broadcast a second data packet, where the second data packet contains second identification information of the first drug infusion device 13; the second identification information may be the media access control address (MAC address, a physical address) information, device serial number information, name information, and model information of the first drug infusion device 13;
the control device 11 is configured to receive the second data packet, obtain the contained second identification information, and send the second identification information to the server 14 for verification; the process of verifying the second identification information by the server 14 is to determine whether the server 14 is connected to the first drug infusion device 13 based on the second identification information; if yes, the verification is passed, and if no, the verification fails;
the server 14 is configured to return first authentication information to the control device 11 after the verification is passed;
the control device 11 is configured to connect to the first drug infusion device 13 and perform mutual authentication based on the first authentication information, generate a fourth shared key after successful authentication, and activate the first drug infusion device 13;
the first drug infusion device 13 is configured to generate the fourth shared key and return an activation success notification.

The process of connecting the control device 11 to the first drug infusion device 13 and performing mutual authentication based on the first authentication information is described below:
the control device 11 generates a first public key and a first private key by using a preset key agreement algorithm; selects any one of the first keys from the key set in the first authentication information, and generates first signature information by using a Hash algorithm based on the selected first key, first time information, and the first public key; sends a first authentication request to the first drug infusion device 13, where the first authentication request contains identifier information of the first key, the first time information, the first public key, and the first signature information, so that the first drug infusion device 13 generates second signature information by using the Hash algorithm based on the key corresponding to the identifier of the first key, the first time information, and the first public key to verify whether the second signature information is consistent with the first signature information;
the control device 11 receives a second authentication request from the first drug infusion device when determining that the second signature information is consistent with the first signature information, where the second authentication request contains identifier information of a second key, second time information, a second public key, and third signature information, the third signature information is generated by the first drug infusion device 13 by using the Hash algorithm based on the selected second key, the second time information and the second public key, and the second public key is generated by the first drug infusion device 13 by using the preset key agreement algorithm; generates fourth signature information by using the Hash algorithm based on the key corresponding to the identifier of the second key, the second time information, and the second public key; determines that the authentication is successful if determining that the fourth signature information is consistent with the third signature information.

The control device 11 generates a fourth shared key after successful mutual authentication with the first drug infusion device 13, and activates the first drug infusion device 13; the first drug infusion device 13 generates the fourth shared key and returns an activation success notification. In the subsequent data interaction between the control device 11 and the first drug infusion device 13, the data is encrypted and decrypted with the fourth shared key to ensure data security. It should be noted that the data sent from the first drug infusion device 13 to the control device 11 may be encrypted with the fourth shared key, and the control device 11 decrypts the encrypted data with the fourth shared key. The data sent from the control device 11 to the first drug infusion device 13 may also be encrypted with the fourth shared key, and the first drug infusion device 13 decrypts the encrypted data with the fourth shared key. Alternatively, the first drug infusion device 13 and the control device 11 may encrypt the sent data with different shared keys.

In the present application, the control device 11 is configured to awaken the first health monitoring device 12 through NFC;
the first health monitoring device 12 is configured to broadcast a third data packet, where the third data packet contains first identification information of the first health monitoring device;
the control device 11 is configured to receive the third data packet, obtain the contained first identification information, and send the first identification information to the server 14 for verification; the process of verifying the first identification information by the server is to determine whether the server 14 is connected to the first health monitoring device 12 based on the first identification information; if yes, the verification is passed, and if no, the verification fails;
the server 14 is configured to return second authentication information to the control device 11 after the verification is passed;
the control device 11 is configured to connect to the first health monitoring device 12 and perform mutual authentication based on the second authentication information, generate a fifth shared key after successful authentication, and activate the first health monitoring device 12;
the first health monitoring device 12 is configured to generate the fifth shared key and return an activation success notification.

The process of mutual authentication between the control device 11 and the first health monitoring device 12 based on the second authentication information is described below:
the control device 11 generates a public key and a private key by using a preset key agreement algorithm; selects any one of the keys from the key set in the second authentication information, and generates signature information by using a Hash algorithm based on the selected key, time information, and the public key; sends an authentication request to the first health monitoring device 12, where the authentication request contains identifier information of the key, the time information, the public key, and the signature information, so that the first health monitoring device 12 generates signature information by using the Hash algorithm based on the key corresponding to the identifier of the key, the time information, and the public key to verify whether the two signature information are consistent;
the control device 11 receives an authentication request from the first health monitoring device when determining that the two signature information are consistent, where the authentication request contains identifier information of a key, time information, a public key, and signature information, the signature information is generated by the first health monitoring device 12 by using the Hash algorithm based on the selected key, the time information and the public key, and the public key is generated by the first health monitoring device 12 by using the preset key agreement algorithm; generates signature information by using the Hash algorithm based on the key corresponding to the identifier of the key, the time information, and the public key; determines that the authentication is successful if determining that the two signature information are consistent.

The control device 11 generates a fifth shared key after successful mutual authentication with the first health monitoring device 12, and activates the first health monitoring device 12; the first health monitoring device 12 generates the fifth shared key and returns an activation success notification. In the subsequent data interaction between the control device 11 and the first health monitoring device 12, the data is encrypted and decrypted with the fifth shared key to ensure data security. It should be noted that the data sent from the first health monitoring device 12 to the control device 11 may be encrypted with the fifth shared key, and the control device 11 decrypts the encrypted data with the fifth shared key. The data sent from the control device 11 to the first health monitoring device 12 may also be encrypted with the fifth shared key, and the first health monitoring device 12 decrypts the encrypted data with the fifth shared key. Alternatively, the first health monitoring device 12 and the control device 11 may encrypt the sent data with different shared keys.

In the present application, the control device 11 is configured to randomly generate third authentication information, encrypt the third authentication information with the fifth shared key, and send the encrypted third authentication information to the first health monitoring device 12;
the first health monitoring device 12 is configured to decrypt the encrypted information with the fifth shared key to obtain the third authentication information;
the control device 11 disconnects from the first health monitoring device 12;
the control device 11 encrypts the first identification information and the third authentication information with the fourth shared key and sends them to the first drug infusion device 13;
the first drug infusion device 13 is configured to decrypt the encrypted information with the fourth shared key to obtain the third authentication information and the first identification information;
the first health monitoring device 12 is configured to broadcast a fourth data packet, where the fourth data packet contains first identification information of the first health monitoring device;
the first drug infusion device 13 is configured to scan a connectable health monitoring device, match with the first health monitoring device based on the first identification information, and establish a connection to the first health monitoring device 12;
the first drug infusion device 13 is configured to perform mutual authentication with the first health monitoring device 12 based on the third authentication information, generate a sixth shared key after successful authentication, and return first state information indicating the successful binding of the first health monitoring device 12 to the control device 11.

The process of mutual authentication between the first drug infusion device 13 and the first health monitoring device 12 based on the third authentication information is described below:
the first health monitoring device 12 generates a public key and a private key by using a preset key agreement algorithm; selects any one of the keys from the key set in the third authentication information, and generates signature information by using a Hash algorithm based on the selected key, time information, and the public key; sends an authentication request to the first drug infusion device 13, where the authentication request contains identifier information of the key, the time information, the public key, and the signature information, so that the first drug infusion device 13 generates signature information by using the Hash algorithm based on the key corresponding to the identifier of the key, the time information, and the public key to verify whether the two signature information are consistent;
the first health monitoring device 12 receives an authentication request from the first drug infusion device 13 when determining that the two signature information are consistent, where the authentication request contains identifier information of a key, time information, a public key, and signature information, the signature information is generated by the first drug infusion device 13 by using the Hash algorithm based on the selected key, the time information and the public key, and the public key is generated by the first drug infusion device 13 by using the preset key agreement algorithm; generates signature information by using the Hash algorithm based on the key corresponding to the identifier of the key, the time information, and the public key; determines that the authentication is successful if determining that the two signature information are consistent.

The first drug infusion device 13 generates a sixth shared key after successful mutual authentication with the first health monitoring device 12. In the subsequent data interaction between the first drug infusion device 13 and the first health monitoring device 12, the data is encrypted and decrypted with the sixth shared key to ensure data security. It should be noted that the data sent from the first health monitoring device 12 to the first drug infusion device 13 may be encrypted with the sixth shared key, and the first drug infusion device decrypts the encrypted data with the sixth shared key. The data sent from the first drug infusion device 13 to the first health monitoring device 12 may also be encrypted with the sixth shared key, and the first health monitoring device 12 decrypts the encrypted data with the sixth shared key. Alternatively, the first health monitoring device 12 and the first drug infusion device 13 may encrypt the sent data with different shared keys.

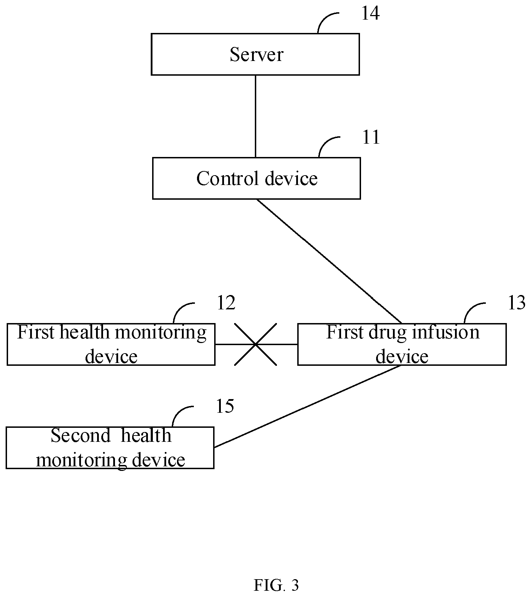

In the present application, it is considered that the first health monitoring device 12 has a validity period, and the validity period is generally one day or two days. When the validity period of the first health monitoring device 12 expires, the first health monitoring device 12 fails and disconnects from the first drug infusion device 13. To replace the second health monitoring device 15 when the first health monitoring device 12 fails, health monitoring is continued for the first user, the system also includes: the second health monitoring device 15. FIG. 3 is a structural diagram of a health monitoring system according to the present application.
the first drug infusion device 13 is further configured to disconnect from the first health monitoring device 12 when identifying that the first health monitoring device 12 expires and fails, and send second state information indicating the disconnection from the first health monitoring device 12 and third state information indicating the expiration of the first health monitoring device 12to the control device;
the control device 11 is further configured to awaken the second health monitoring device 15 through NFC after receiving the second state information and the third state information;
the second health monitoring device 15 is configured to broadcast a fifth data packet, where the fifth data packet contains third identification information of the second health monitoring device;
the control device 11 is configured to receive the fifth data packet, obtain the contained third identification information, connect to the second health monitoring device 15 for mutual authentication, activate the second health monitoring device 15, and send the third identification information to the first drug infusion device 13;
the control device 11 is configured to randomly generate fourth authentication information, encrypt the fourth authentication information, and send the encrypted fourth authentication information to the second health monitoring device 15 and the first drug infusion device 13;
the first drug infusion device 13 is configured to scan a connectable health monitoring device, match with the second health monitoring device 15 based on the third identification information, and connect to the second health monitoring device 15 for mutual authentication based on the fourth authentication information.

The process of connecting the first drug infusion device 13 to the second health monitoring device 15 for mutual authentication based on the fourth authentication information is described below:
the first drug infusion device 13 generates a public key and a private key by using a preset key agreement algorithm; selects any one of the keys from the key set in the fourth authentication information, and generates signature information by using a Hash algorithm based on the selected key, time information, and the public key; sends an authentication request to the second health monitoring device 15, where the authentication request contains identifier information of the key, the time information, the public key, and the signature information, so that the second health monitoring device 15 generates signature information by using the Hash algorithm based on the key corresponding to the identifier of the key, the time information, and the public key to verify whether the two signature information are consistent;
the first drug infusion device 13 receives an authentication request from the second health monitoring device 15 when determining that the two signature information are consistent, where the authentication request contains identifier information of a key, time information, a public key, and signature information, the signature information is generated by the second health monitoring device 15 by using the Hash algorithm based on the selected key, the time information and the public key, and the public key is generated by the second health monitoring device by using the preset key agreement algorithm; generates signature information by using the Hash algorithm based on the key corresponding to the identifier of the key, the time information, and the public key; determines that the authentication is successful if determining that the two signature information are consistent.

After the first health monitoring device 12 fails, the connection function of the first health monitoring device 12 is abnormal. At this time, the first drug infusion device 13 connected to the first health monitoring device 12 may identify the disconnection from the first health monitoring device 12. At this time, the first drug infusion device 13 sends second state information indicating the disconnection from the first health monitoring device 12 and third state information indicating the expiration of the first health monitoring device 12 to the control device 11. The control device 11 awakens the second health monitoring device 15 through NFC after it receives the second state information and the third state information and the first user wears the second health monitoring device 15.

After the control device 11 activates the second health monitoring device 15, the second health monitoring device 15 broadcasts a fifth data packet, where the fifth data packet contains third identification information of the second health monitoring device 15; the third identification information may be the MAC address information, device serial number information, name information, and model information of the second health monitoring device 15.

The control device 11 receives the fifth data packet, obtains the contained third identification information, and sends the third identification information to the first drug infusion device 13; the first drug infusion device 13 scans a connectable health monitoring device, matches with the second health monitoring device 15 based on the third identification information, connects to the second health monitoring device 15 for mutual authentication, and activates the second health monitoring device 15.

After the first drug infusion device 13 connects to the second health monitoring device, the second health monitoring device 15 obtains target health monitoring data of a first user and sends the target health monitoring data to the first drug infusion device 13; the first drug infusion device 13 sends the target health monitoring data to the control device 11, receives a drug infusion index corresponding to the target health monitoring data from the control device 11, and infuses the drug to the first user based on the drug infusion index.

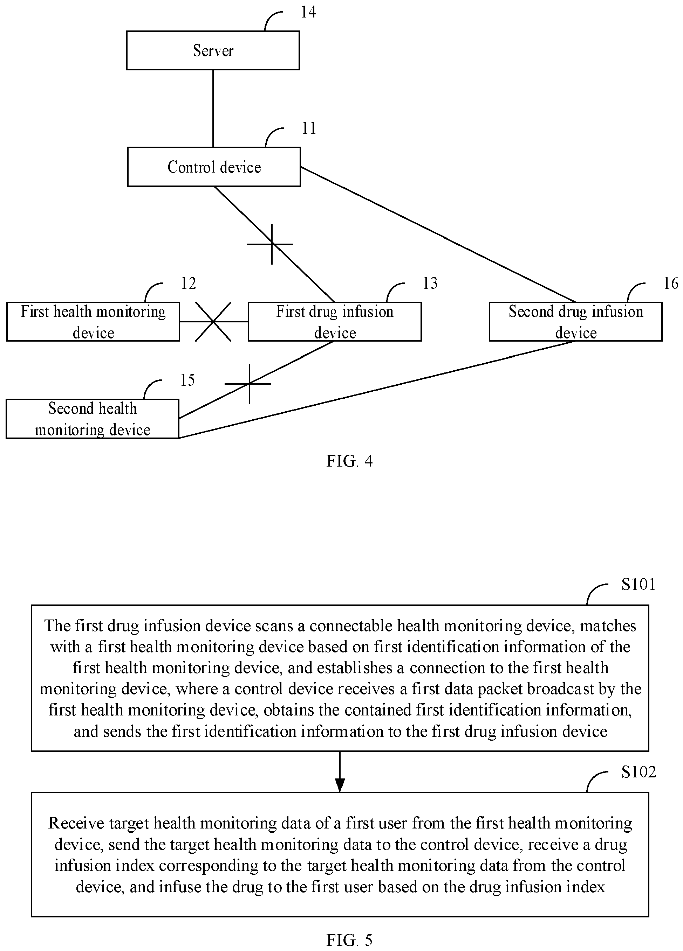

In the present application, it is considered that the first drug infusion device 13 has a validity period, and the validity period is generally one day or two days. When the validity period of the first drug infusion device 13 expires, the first drug infusion device 13 fails and disconnects from the control device 11 and the health monitoring device 15. To replace the second drug infusion device 16 when the first drug infusion device 13 fails, health monitoring is continued for the first user, and the system further includes a second drug infusion device 16FIG. 4 is a structural diagram of a health monitoring system according to the present application.
the control device 11 is further configured to connect to a recently activated third health monitoring device when identifying that the first drug infusion device 13 expires and fails and the second drug infusion device 16 is not detected, to obtain health monitoring data collected by the third health monitoring device;
the control device 11 is further configured to awaken the second drug infusion device 16 through NFC, perform mutual authentication and activate the second drug infusion device 16 when detecting the second drug infusion device 16, and send fourth identification information of the third health monitoring device to the second drug infusion device 16;
the control device 11 is configured to randomly generate fifth authentication information, encrypt the fifth authentication information, and send the encrypted fifth authentication information to the third health monitoring device and the second drug infusion device 16;
the second drug infusion device 16 is configured to scan a connectable health monitoring device, match with the third health monitoring device based on the fourth identification information, and connect to the third health monitoring device for mutual authentication based on the fifth authentication information.

If the first drug infusion device 13 expires and fails and the first user does not wear the second drug infusion device 16, the control device 11 connects to the recently activated third health monitoring device. If the first health monitoring device 12 is activated recently, the first health monitoring device 12 is used as the third health monitoring device, and if the second health monitoring device 15 is activated recently, the second health monitoring device 15 is used as the third health monitoring device. The third health monitoring device sends the collected health monitoring data to the control device 11, and the control device 11 obtains the health monitoring data collected by the third health monitoring device.

If the first drug infusion device 13 expires and fails and the first user wears the second drug infusion device 16, the control device 11 awakens the second drug infusion device 16 through NFC, performs mutual authentication and activates the second drug infusion device 16; the control device 11 sends the fourth identification information of the third health monitoring device to the second drug infusion device 16. The second drug infusion device 16 scans a connectable health monitoring device, matches with the third health monitoring device based on the fourth identification information, and connects to the third health monitoring device for mutual authentication based on the fifth authentication information.

The process of connecting the second drug infusion device 16 to the third health monitoring device for mutual authentication based on the fifth authentication information is described below:
the second drug infusion device 16 generates a public key and a private key by using a preset key agreement algorithm; selects any one of the keys from the key set in the fifth authentication information, and generates signature information by using a Hash algorithm based on the selected key, time information, and the public key; sends an authentication request to the third health monitoring device, where the authentication request contains identifier information of the key, the time information, the public key, and the signature information, the third health monitoring device generates signature information by using the Hash algorithm based on the key corresponding to the identifier of the key, the time information, and the public key to verify whether the two signature information are consistent;
the second drug infusion device 16 receives an authentication request from the third health monitoring device when determining that the two signature information are consistent, where the authentication request contains identifier information of a key, time information, a public key, and signature information, the signature information is generated by the third health monitoring device by using the Hash algorithm based on the selected key, the time information and the public key, and the public key is generated by the third health monitoring device by using the preset key agreement algorithm; generates signature information by using the Hash algorithm based on the key corresponding to the identifier of the key, the time information, and the public key; determines that the authentication is successful if determining that the two signature information are consistent.

After the first drug infusion device 13 fails, the connection function of the first drug infusion device 13 is abnormal. At this time, the control device 11 connected to the first drug infusion device 13 may identify the disconnection from the first drug infusion device 13. At this time, after the first user wears the second drug infusion device 16, the control device 11 activates the second drug infusion device 16. The control device 11 may awaken the second drug infusion device 16 through NFC and activate the second drug infusion device 16.

The control device 11 obtains second identification information of the recently activated health monitoring device after activating the second drug infusion device. The recently activated health monitoring device may be the first health monitoring device 12 or the second health monitoring device 15. FIG. 4 illustrates an example in which the recently activated health monitoring device is the second health monitoring device 15. The fourth identification information of the recently activated health monitoring device includes media access control address (MAC address, a physical address) information, device serial number information, name information, and model information. The control device 11 sends the fourth identification information of the recently activated health monitoring device to the second drug infusion device 16, and the second drug infusion device 16 may search for the recently activated health monitoring device based on the second identification information and establish a Bluetooth connection to the recently activated health monitoring device.

After the second drug infusion device 16 connects to the recently activated third health monitoring device, the third health monitoring device obtains target health monitoring data of a first user and sends the target health monitoring data to the second drug infusion device 16; the second drug infusion device 16 sends the target health monitoring data to the control device 11, receives a drug infusion index corresponding to the target health monitoring data from the control device 11, and infuses the drug to the first user based on the drug infusion index. The control device 11 adjusts the drug infusion index in real time according to the preset drug infusion rules.

In the present application, the control device 11 is further configured to disconnect from a third drug infusion device when detecting itself away from the third drug infusion device and a fourth health monitoring device in a connected state;
the third drug infusion device is configured to receive health monitoring data from the fourth health monitoring device after detecting the disconnection from the control device 11, determine a drug infusion index corresponding to the health monitoring data, and infuse the drug to the first user based on the drug infusion index.

The third drug infusion device in a connected state may be the first drug infusion device 13 or the second drug infusion device 16, and the fourth health monitoring device in a connected state may be the first health monitoring device 12 or the second health monitoring device 15.

The control device 11 has the highest priority. If the control device 11 does not disconnect, the drug infusion device needs to send health monitoring data to the control device 11. The control device 11 determines a drug infusion index and sends the drug infusion index to the drug infusion device, and then the drug infusion device infuses the drug to the user based on the drug infusion index. When the control device 11 disconnects, the drug infusion device has the capability of determining a corresponding drug infusion index based on the health monitoring data. At this time, after determining the drug infusion index, the drug infusion device infuses the drug to the user based on the drug infusion index.

The third drug infusion device is further configured to broadcast a sixth data packet after the disconnection from the control device 11, and the sixth data packet contains fifth identification information of the third drug infusion device;
the control device 11 is further configured to scan a connectable drug infusion device, match with the third drug infusion device based on the fifth identification information, and establish a connection to the third drug infusion device;
the third drug infusion device is configured to receive health monitoring data from the fourth health monitoring device after detecting that a connection to the control device 11 is established and the mutual authentication is successful, send the health monitoring data to the control device 11, receive a drug infusion index corresponding to the health monitoring data from the control device 11, and infuse the drug to the first user based on the drug infusion index.

FIG. 5 is a schematic diagram of a health monitoring process according to the present application, where the process is applied to a first drug infusion device 13 and includes the following steps:
S 1 01: The first drug infusion device 13 scans a connectable health monitoring device, matches with a first health monitoring device 12 based on first identification information of the first health monitoring device 12, and establishes a connection to the first health monitoring device 12, where a control device 11 receives a first data packet broadcast by the first health monitoring device 12, obtains the contained first identification information, and sends the first identification information to the first drug infusion device 13.
S 102: The first drug infusion device receives target health monitoring data of a first user from the first health monitoring device 12, sends the target health monitoring data to the control device 11, receives a drug infusion index corresponding to the target health monitoring data from the control device 11, and infuses the drug to the first user based on the drug infusion index.

Optionally, the first drug infusion device 13 receives target health monitoring data of a first user encrypted with a first shared key from the first health monitoring device 12;
the first drug infusion device 13 decrypts the target health monitoring data with the first shared key, encrypts the decrypted data with a second shared key, and sends the data encrypted with the second shared key to the control device 11, s the control device 11 decrypts the data with the second shared key after receiving the data to obtain the target health monitoring data; the control device 11 sends a drug infusion index corresponding to the target health monitoring data encrypted with a third shared key to the first drug infusion device; the first drug infusion device decrypts the data with the third shared key to obtain the drug infusion index, and infuses the drug to the first user based on the drug infusion index.

Optionally, the first drug infusion device 13 broadcasts a second data packet, where the second data packet contains second identification information of the first drug infusion device; the control device 11 receives the second data packet, obtains the contained second identification information, and sends the second identification information to the server 14 for verification; the server 14 returns first authentication information to the control device 11 after the verification is passed; the control device 11 connects to the first drug infusion device 13 and performs mutual authentication based on the first authentication information, generates a fourth shared key after successful authentication, and activates the first drug infusion device 13;
the first drug infusion device 13 generates the fourth shared key and returns an activation success notification.

The first drug infusion device 13 decrypts the encrypted information with the fourth shared key to obtain third authentication information and the first identification information;
the first drug infusion device 13 scans a connectable health monitoring device, matches with the first health monitoring device 12 based on the first identification information, and establishes a connection to the first health monitoring device 12;
the first drug infusion device 13 performs mutual authentication with the first health monitoring 12 device based on the third authentication information, generates a sixth shared key after successful authentication, and returns first state information indicating the successful binding of the first health monitoring device 12 to the control device 11.

The first drug infusion device 13 disconnects from the first health monitoring device 12 when identifying that the first health monitoring device 12 expires and fails, and sends second state information indicating the disconnection from the first health monitoring device 12 and third state information indicating the expiration of the first health monitoring device 12 to the control device 11; the control device 11 awakens the second health monitoring device 15 through NFC after receiving the second state information and the third state information; the second health monitoring device 15 broadcasts a fifth data packet, where the fifth data packet contains third identification information of the second health monitoring device 15; the control device 11 receives the fifth data packet, obtains the contained third identification information, connects to the second health monitoring device 15 for mutual authentication, activates the second health monitoring device 15, and sends the third identification information to the first drug infusion device 13;
the first drug infusion device 13 scans a connectable health monitoring device, matches with the second health monitoring device 15 based on the third identification information, and connects to the second health monitoring device 15 for mutual authentication based on the fourth authentication information.

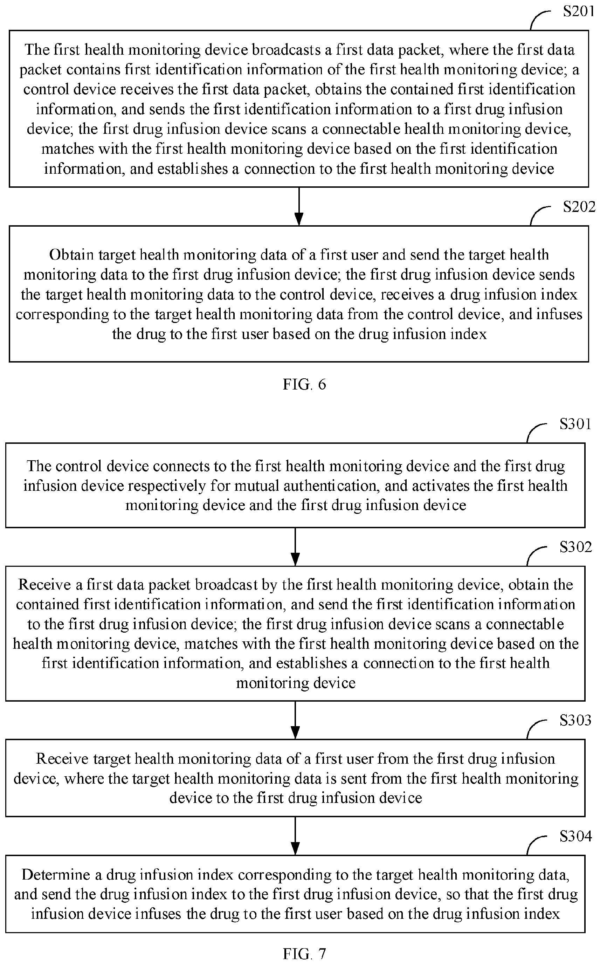

FIG. 6 is a schematic diagram of a health monitoring process according to the present application, where the process is applied to a first health monitoring device 12 and includes the following steps:
S201: The first health monitoring device 12 broadcasts a first data packet, where the first data packet contains first identification information of the first health monitoring device 12; a control device 11 receives the first data packet, obtains the contained first identification information, and sends the first identification information to a first drug infusion device 13; the first drug infusion device 13 scans a connectable health monitoring device, matches with the first health monitoring device based on the first identification information, and establishes a connection to the first health monitoring device 12.
S202: The first health monitoring device 12 obtains target health monitoring data of a first user and sends the target health monitoring data to the first drug infusion device 13; the first drug infusion device 13 sends the target health monitoring data to the control device 11, receives a drug infusion index corresponding to the target health monitoring data from the control device 11, and infuses the drug to the first user based on the drug infusion index.

Optionally, the first health monitoring device 12 obtains target health monitoring data of a first user, encrypts the target health monitoring data with a first shared key, and sends the encrypted target health monitoring data to the first drug infusion device 13; the first drug infusion device 13 decrypts the target health monitoring data with the first shared key, encrypts the decrypted data with a second shared key, and sends the data encrypted with the second shared key to the control device 11; the control device 11 decrypts the data with the second shared key after receiving the data to obtain the target health monitoring data; the first drug infusion device 13 receives a drug infusion index corresponding to the target health monitoring data encrypted with a third shared key from the control device 11, decrypts the data with the third shared key to obtain the drug infusion index, and infuses the drug to the first user based on the drug infusion index.

The first health monitoring device 12 broadcasts a third data packet, where the third data packet contains first identification information of the first health monitoring device 12; the control device 11 receives the third data packet, obtains the contained first identification information, and sends the first identification information to the server 14 for verification; the server 14 returns second authentication information to the control device 11 after the verification is passed; the control device 11 connects to the first health monitoring device 12 and performs mutual authentication based on the second authentication information, generates a fifth shared key after successful authentication, and activates the first health monitoring device 12;
the first health monitoring device 12 generates the fifth shared key and returns an activation success notification.

The first health monitoring device 12 decrypts the encrypted information with the fifth shared key to obtain third authentication information, where the control device 11 randomly generates the third authentication information, encrypts the third authentication information with the fifth shared key, and sends the encrypted third authentication information to the first health monitoring device 12;
the first health monitoring device 12 broadcasts a fourth data packet, where the fourth data packet contains first identification information of the first health monitoring device 12; the control device 11 receives the fourth data packet and obtains the contained first identification information; the first drug infusion device 13 scans a connectable health monitoring device, matches with the first health monitoring device 12 based on the first identification information, and establishes a connection to the first health monitoring device 12; the first drug infusion device 13 performs mutual authentication with the first health monitoring device 12 based on the third authentication information, generates a sixth shared key after successful authentication, and returns first state information indicating the successful binding of the first health monitoring device 12 to the control device 11.

FIG. 7 is a schematic diagram of a health monitoring process according to the present application, where the process is applied to a control device 11 and includes the following steps:
S301: The control device 11 connects to the first health monitoring device 12 and the first drug infusion device 13 respectively for mutual authentication, and activates the first health monitoring device 12 and the first drug infusion device 13.
S302: The control device 11 receives a first data packet broadcast by the first health monitoring device 12, obtains the contained first identification information, and sends the first identification information to the first drug infusion device 13; the first drug infusion device 13 scans a connectable health monitoring device, matches with the first health monitoring device 12 based on the first identification information, and establishes a connection to the first health monitoring device 12.
S303: The control device 11 receives target health monitoring data of a first user from the first drug infusion device, where the target health monitoring data is sent from the first health monitoring device 12 to the first drug infusion device 13.
S304: The control device 11 determines a drug infusion index corresponding to the target health monitoring data, and sends the drug infusion index to the first drug infusion device 13, the first drug infusion device 13 infuses the drug to the first user based on the drug infusion index.

The control device 11 receives the decrypted data encrypted by the first drug infusion device 13 with a second shared key, where the first health monitoring device 12 obtains target health monitoring data of a first user, encrypts the target health monitoring data with a first shared key, and sends the encrypted target health monitoring data to the first drug infusion device 13; the first drug infusion device 13 decrypts the target health monitoring data with the first shared key and encrypts the decrypted data with the second shared key;
the control device 11 decrypts the data with the second shared key to obtain the target health monitoring data, encrypts a drug infusion index corresponding to the target health monitoring data with a third shared key, and sends the drug infusion index to the first drug infusion device 13, so that the first drug infusion device 13 decrypts the data with the third shared key to obtain the drug infusion index, and infuses the drug to the first user based on the drug infusion index.

The control device 11 awakens the first drug infusion device 13 through NFC;
the control device 11 receives a second data packet broadcast by the first drug infusion device 13, obtains the contained second identification information, sends the second identification information to the server 14 for verification, and receives first authentication information returned by the server 14 after the verification is passed;
the control device 11 connects to the first drug infusion device 13 and performs mutual authentication based on the first authentication information, generates a fourth shared key after successful authentication, and activates the first drug infusion device 13; the first drug infusion device 13 generates the fourth shared key and returns an activation success notification.

The control device 11 awakens the first health monitoring device 12 through NFC;
the control device receives a third data packet broadcast by the first health monitoring device 12, obtains the contained first identification information, sends the first identification information to the server 14 for verification, and receives second authentication information returned by the server 14 after the verification is passed;
the control device 11 connects to the first health monitoring device 12 and performs mutual authentication based on the second authentication information, generates a fifth shared key after successful authentication, and activates the first health monitoring device 12; the first health monitoring device 12 generates the fifth shared key and returns an activation success notification.

The control device 11 randomly generates third authentication information, encrypts the third authentication information with the fifth shared key, and sends the encrypted third authentication information to the first health monitoring device 12; the first health monitoring device 12 decrypts the encrypted information with the fifth shared key to obtain the third authentication information;
the control device 11 disconnects from the first health monitoring device 12;
the control device 11 encrypts the first identification information and the third authentication information with the fourth shared key and sends them to the first drug infusion device 13; the first drug infusion device 13 decrypts the encrypted information with the fourth shared key to obtain the third authentication information and the first identification information; the first health monitoring device 12 broadcasts a fourth data packet, where the fourth data packet contains first identification information of the first health monitoring device 12; the first drug infusion device 13 scans a connectable health monitoring device, matches with the first health monitoring device 12 based on the first identification information, and establishes a connection to the first health monitoring device 12; the first drug infusion device 13 performs mutual authentication with the first health monitoring device 12 based on the third authentication information, generates a sixth shared key after successful authentication, and returns first state information indicating the successful binding of the first health monitoring device 12 to the control device 11.

The control device 11 awakens the second health monitoring device 15 through NFC after receiving second state information and third state information, where the first drug infusion device 13 disconnects from the first health monitoring device 12 when identifying that the first health monitoring device 12 expires and fails, and sends the second state information indicating the disconnection from the first health monitoring device 12 and the third state information indicating the expiration of the first health monitoring device 12 to the control device 11;
the control device 11 receives a fifth data packet broadcast by the second health monitoring device 15, obtains the contained third identification information, connects to the second health monitoring device 15 for mutual authentication, activates the second health monitoring device 15, and sends the third identification information to the first drug infusion device 13; randomly generates fourth authentication information, encrypts the fourth authentication information, and sends the encrypted fourth authentication information to the second health monitoring device 15 and the first drug infusion device 13; the first drug infusion device 13 scans a connectable health monitoring device, matches with the second health monitoring device 15 based on the third identification information, and connects to the second health monitoring device 15 for mutual authentication based on the fourth authentication information.

The control device 11 connects to a recently activated third health monitoring device when identifying that the first drug infusion device 13 expires and fails and the second drug infusion device 16 is not detected, to obtain health monitoring data collected by the third health monitoring device;
the control device 11 awakens the second drug infusion device 16 through NFC, performs mutual authentication and activates the second drug infusion device 16 when detecting the second drug infusion device 16, and sends fourth identification information of the third health monitoring device to the second drug infusion device 16; randomly generates fifth authentication information, encrypts the fifth authentication information, and sends the encrypted fifth authentication information to the third health monitoring device and the second drug infusion device 16; the second drug infusion device 16 scans a connectable health monitoring device, matches with the third health monitoring device based on the fourth identification information, and connects to the third health monitoring device for mutual authentication based on the fifth authentication information.

The control device 11 disconnects from a third drug infusion device when detecting itself away from the third drug infusion device and a fourth health monitoring device in a connected state; the third drug infusion device receives health monitoring data from the fourth health monitoring device after detecting the disconnection from the control device 11, determines a drug infusion index corresponding to the health monitoring data, and infuses the drug to the first user based on the drug infusion index.

The control device 11 scans a connectable drug infusion device, matches with the third drug infusion device based on fifth identification information, and establishes a connection to the third drug infusion device, where the third drug infusion device broadcasts a sixth data packet after the disconnection from the control device, and the sixth data packet contains the fifth identification information of the third drug infusion device; the third drug infusion device receives health monitoring data from the fourth health monitoring device after detecting that a connection to the control device 11 is established and the mutual authentication is successful, sends the health monitoring data to the control device 11, receives a drug infusion index corresponding to the health monitoring data from the control device 11, and infuses the drug to the first user based on the drug infusion index.

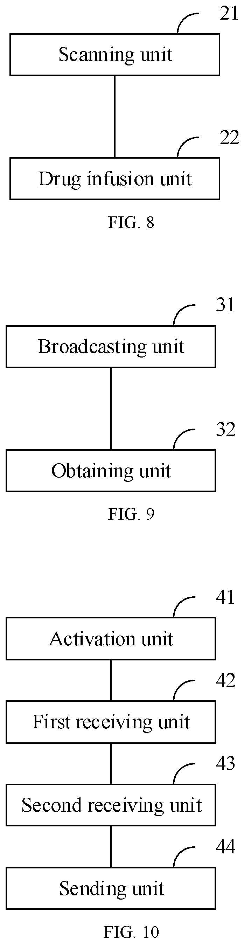

FIG. 8 is a structural diagram of a health monitoring apparatus according to the present application, where the apparatus includes:
a scanning unit 21, configured to scan a connectable health monitoring device, match with a first health monitoring device 12 based on first identification information of the first health monitoring device 12, and establish a connection to the first health monitoring device 12, where a control device 11 receives a first data packet broadcast by the first health monitoring device 12, obtains the contained first identification information, and sends the first identification information to the first drug infusion device 13 through the control device; and
a drug infusion unit 22, configured to receive target health monitoring data of a first user from the first health monitoring device 12, send the target health monitoring data to the control device 11, receive a drug infusion index corresponding to the target health monitoring data from the control device 11, and infuse the drug to the first user based on the drug infusion index.

FIG. 9 is a structural diagram of a health monitoring apparatus according to the present application, where the apparatus includes:
a broadcasting unit 31, configured to broadcast a first data packet, where the first data packet contains first identification information of the first health monitoring device 12; a control device 11 receives the first data packet, obtains the contained first identification information, and sends the first identification information to a first drug infusion device 13; the first drug infusion device 13 scans a connectable health monitoring device, matches with the first health monitoring device 12 based on the first identification information, and establishes a connection to the first health monitoring device 12;
an obtaining unit 32, configured to obtain target health monitoring data of a first user and send the target health monitoring data to the first drug infusion device 13; the first drug infusion device 13 sends the target health monitoring data to the control device 11, receives a drug infusion index corresponding to the target health monitoring data from the control device 11, and infuses the drug to the first user based on the drug infusion index.

FIG. 10 is a structural diagram of a health monitoring apparatus according to the present application, where the apparatus includes:
an activation unit 41, configured to connect to the first health monitoring device 12 and the first drug infusion device 13 respectively for mutual authentication, and activate the first health monitoring device 12 and the first drug infusion device 13;
a first receiving unit 42, configured to receive a first data packet broadcast by the first health monitoring device 12, obtain the contained first identification information, and send the first identification information to the first drug infusion device 13; the first drug infusion device 13 scans a connectable health monitoring device, matches with the first health monitoring device 12 based on the first identification information, and establishes a connection to the first health monitoring device 12;
a second receiving unit 43, configured to receive target health monitoring data of a first user from the first drug infusion device 13, where the target health monitoring data is sent from the first health monitoring device 12 to the first drug infusion device 13;
a sending unit 44, configured to determine a drug infusion index corresponding to the target health monitoring data, and send the drug infusion index to the first drug infusion device 13, the first drug infusion device 13 infuses the drug to the first user based on the drug infusion index.

In the present application, the control device can connect to the drug infusion device through Bluetooth Low Energy, and the drug infusion device can connect to the health monitoring device through Bluetooth Low Energy. By obtaining health monitoring data, the control device can implement a guidance algorithm to control and adjust the drug infusion device. When the control device disconnects, the drug infusion device can remain connected to the health monitoring device and makes adjustments based on the health monitoring data.

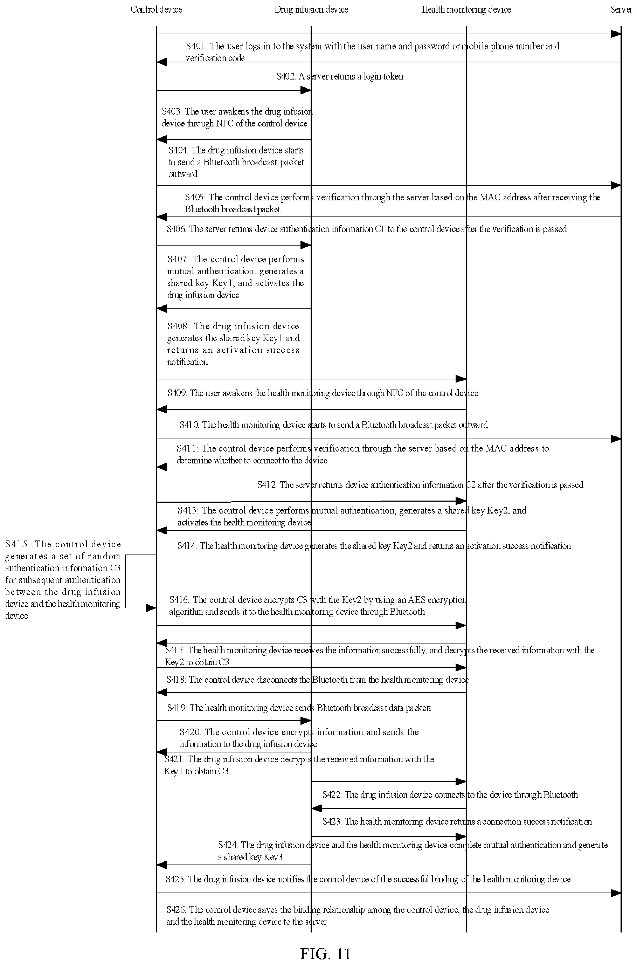

FIG. 11 is a flow chart of first activation and use of a drug infusion device and a health monitoring device according to the present application, where the process involves the interaction among a control device, the health monitoring device and the drug infusion device. The process includes the following steps:
S401: the user logs in to the system with the user's name and password or mobile phone number and verification code;
S402: a server returns a login token;
S403: the user awakens the drug infusion device through NFC of the control device;
S404: the drug infusion device starts to send a Bluetooth broadcast packet outward;
S405: the control device performs verification through the server based on the MAC address after receiving the Bluetooth broadcast packet, to determine whether to connect to the device;
S406: the server returns device authentication information C1 to the control device after the verification is passed;
S407: the control device connects to the drug infusion device through Bluetooth, performs mutual authentication based on C1, generates a shared key Key1 after successful authentication, and activates the drug infusion device;
S408: the drug infusion device generates the shared key Key1 and returns an activation success notification;
S409: the user awakens the health monitoring device through NFC of the control device;
S410: the health monitoring device starts to send a Bluetooth broadcast packet outward;
S411: the control device performs verification through the server based on the MAC address after receiving the Bluetooth broadcast packet, to determine whether to connect to the device;
S412: the server returns device authentication information C2 after the verification is passed;
S413: the control device connects to the health monitoring device through Bluetooth, performs mutual authentication based on C2, generates a shared key Key2 after successful authentication, and activates the health monitoring device;
S414: the health monitoring device generates the shared key Key2 and returns an activation success notification;
S415: the control device generates a set of random authentication information C3 for subsequent authentication between the drug infusion device and the health monitoring device;
S416: the control device encrypts C3 with the Key2 by using an AES encryption algorithm and sends it to the health monitoring device through Bluetooth;
S417: the health monitoring device receives the information successfully, and decrypts the received information with the Key2 to obtain C3 and save it;
S418: the control device disconnects the Bluetooth from the health monitoring device;
S419: after successful disconnection of Bluetooth from the health monitoring device, the health monitoring device continuously sends Bluetooth broadcast data packets outward;
S420: the control device encrypts C3 and health monitoring device information (including unique identification and state information of the health monitoring device) with the Key1 by using the AES, and sends them to the drug infusion device;
S421: the drug infusion device receives the information successfully, and decrypts the received information with the Key1 to obtain C3 and save it;
S422: the drug infusion device scans a connectable health monitoring device around, matches with the received information of the health monitoring device, and connects to the device through Bluetooth when the matching is successful;
S423: the health monitoring device returns a connection success notification;
S424: the drug infusion device and the health monitoring device complete mutual authentication based on the authentication information C3, and generate a shared key Key3 for subsequent data transmission;
S425: the drug infusion device notifies the control device of the successful binding of the health monitoring device;
S426: the control device saves the binding relationship among the control device, the drug infusion device and the health monitoring device to the server.

At this point, the control device activates the drug infusion device and the health monitoring device for the first time, and connects to them for use. In this process, the control device only activates the health monitoring device and disconnects from it after activation. No health monitoring data is transmitted between the control device and the health monitoring device. In the data transmission stage, the control device and the drug infusion device encrypt the data with the Key1 as a key of the AES encryption algorithm and transmit the data. The control device and the health monitoring device encrypt the data with the Key2 as a key of the AES encryption algorithm and transmit the data. The health monitoring device and the drug infusion device encrypt the data with the Key3 as a key of the AES encryption algorithm and transmit the data. When either device disconnects, re-authentication is required and a new data transmission key is generated.

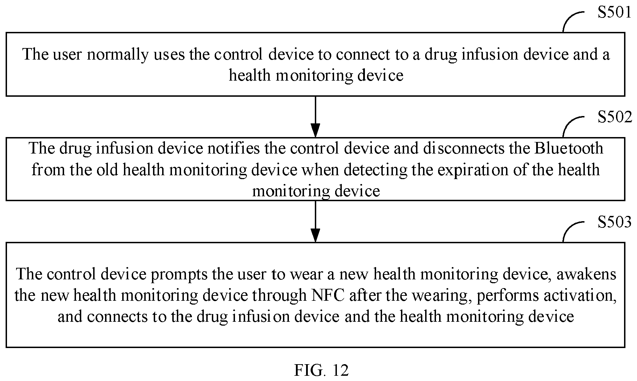

FIG. 12 is a flow chart of replacement of a health monitoring device according to the present application, where the process includes the following steps:
S501: The user normally uses a control device to connect to a drug infusion device and the health monitoring device.
S502: The drug infusion device notifies the control device and disconnects the Bluetooth from the expired health monitoring device when detecting the expiration of the health monitoring device.
S503: The control device prompts the user to wear a new health monitoring device, awakens the new health monitoring device through NFC after the wearing, performs activation, and connects to the drug infusion device and the health monitoring device.
S503 has the same process principles as those of S410-S426 in FIG. 11 and will not be described here.

At this point, the replacement of the health monitoring device is completed.

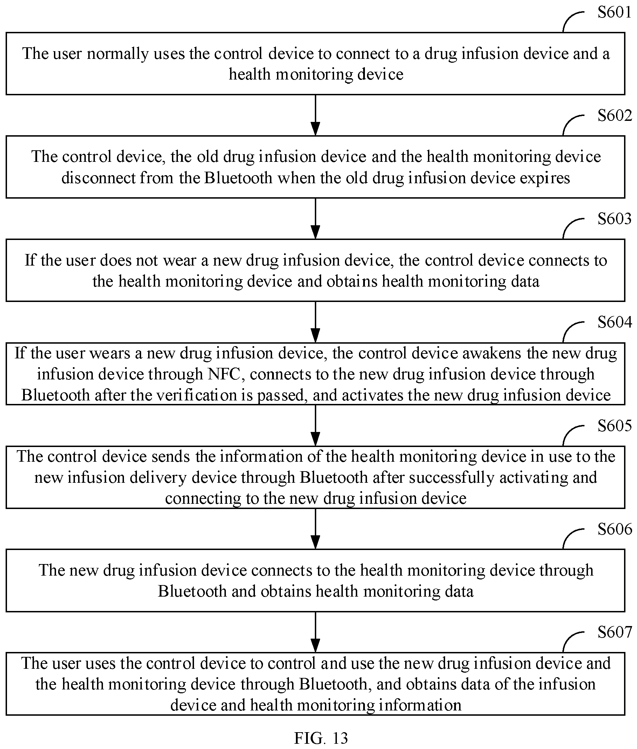

FIG. 13 is a flow chart of replacement of a drug infusion device according to the present application, where the process includes the following steps:
S601: The user normally uses a control device to connect to the drug infusion device and a health monitoring device.
S602: The control device, the expired drug infusion device and the health monitoring device disconnect from the Bluetooth when the expired drug infusion device expires.
S603: If the user does not wear a new drug infusion device, the control device connects (for example, via Bluetooth) to the health monitoring device and obtains health monitoring data.
S604: If the user wears a new drug infusion device, the control device awakens the new drug infusion device through NFC, connects (for example, via Bluetooth) to the new drug infusion device after the verification is passed, and activates the new drug infusion device.
S605: The control device sends (for example, the Bluetooth) the information of the health monitoring device in use to the new drug infusion device after successfully activating and connecting to the new drug infusion device.
S606: The new drug infusion device connects to (for example, the Bluetooth) the health monitoring device and obtains health monitoring data.
S607: The user uses the control device (for example, the Bluetooth) to control and use the new drug infusion device and the health monitoring device, and obtains data of the infusion device and health monitoring information.

The mutual authentication mechanism after the entire connection is established is the same as above. At this point, the replacement of the drug infusion device is completed.

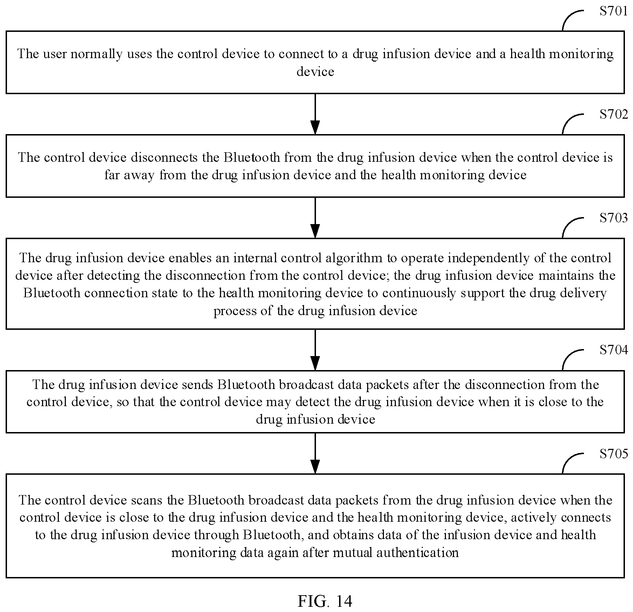

FIG. 14 is a flow chart of reconnection of a control device according to the present application, where the process includes the following steps:
S701: The user normally uses the control device to connect to a drug infusion device and a health monitoring device.
S702: The control device disconnects connection (for example, Bluetooth connection) from the drug infusion device when the control device is far away from the drug infusion device and the health monitoring device.
S703: The drug infusion device enables an internal control algorithm to operate independently of the control device after detecting the disconnection from the control device; the drug infusion device maintains the connection state (for example, Bluetooth connection) to the health monitoring device to continuously support the drug delivery process of the drug infusion device.
S704: The drug infusion device continuously sends broadcast data packets (for example, Bluetooth broadcast data packets) after the disconnection from the control device, so that the control device may detect the drug infusion device when it is close to the drug infusion device.
S705: The control device scans the broadcast data packets (for example, Bluetooth broadcast data packets) from the drug infusion device when the control device is close to the drug infusion device and the health monitoring device, actively connects (for example, Bluetooth connection) to the drug infusion device, and obtains data of the infusion device and health monitoring data again after mutual authentication.

At this point, the control device may automatically restore to the normal connection and use state when it is far away from or close to the device.

In the health monitoring system according to the present application, a control device, a drug infusion device and a health monitoring device are interconnected to form a health monitoring system, and the control device is responsible for the scheduling of the whole system, to realize the security of the whole system, authentication between the devices, and encrypted transmission mechanism. When the drug infusion device or the health monitoring device expires, the control device may autonomously discover and complete the activation of new devices to keep the communication link among them unchanged. When the effective connection range of the control device to the drug infusion device and the health monitoring device changes, the control device may autonomously discover the Bluetooth connection to the completed device, to keep the communication link among them unchanged. The control device has algorithm priority, and may perform intelligent calculations and guide the proper functioning of the drug infusion device. The drug infusion device has the capability to receive, analyze, process, and guide health monitoring data.

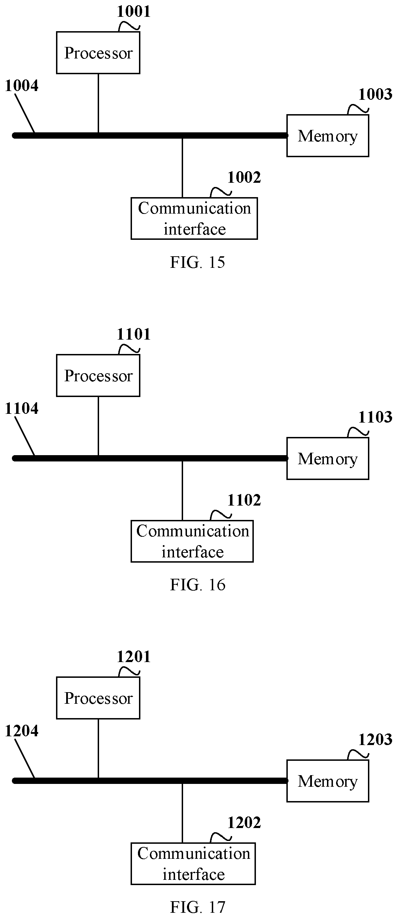

FIG. 15 shows a drug infusion device according to the present application, and the drug fusion device can be the first drug infusion device 13, the second drug infusion device 16 or the third drug infusion device, etc. As shown in FIG. 15, the drug infusion device includes a processor 1001, a communication interface 1002, a memory 1003, and a communication bus 1004, and the processor 1001, the communication interface 1002 and the memory 1003 communicate with each other through the communication bus 1004;
the memory 1003 stores a computer program, and the processor 1001 implements the steps of any of the above methods when the program is executed by the processor 1001.

The communication bus of the above electronic device may be a peripheral component interconnect (PCI) bus or an extended industry standard architecture (EISA) bus. The communication bus may be divided into address bus, data bus, and control bus. For convenience of illustration, the bus is represented only by a thick line in the figure, but it does not mean that there is only one bus or one type of bus.

The communication interface 1002 is used for the communication between the above electronic device and other devices.

The memory may include random access memory (RAM) or non-volatile memory (NVM), for example, it may be at least one disk memory. Alternatively, the memory may also be at least one storage device located remotely from the processor.

The processor may be a general-purpose processor, including central processing unit and network processor (NP), or may also be a digital signal processing (DSP), special integrated circuit, field programmable gate array or programmable logic device, discrete gate or transistor logic device, and discrete hardware component.

FIG. 16 shows a health monitoring device according to the present application, and the drug fusion device can be the first drug infusion device 13, the second drug infusion device 16 or the third drug infusion device, etc. As shown in FIG. 16, the first health monitoring device includes a processor 1101, a communication interface 1102, a memory 1103, and a communication bus 1104, and the processor 1101, the communication interface 1102 and the memory 1103 communicate with each other through the communication bus 1104;
the memory 1103 stores a computer program, and the processor 1101 implements the steps of any of the above methods when the program is executed by the processor 1101.

FIG. 17 shows a control device according to the present application. As shown in FIG. 17, the control device includes a processor 1201, a communication interface 1202, a memory 1203, and a communication bus 1204, and the processor 1201, the communication interface 1202 and the memory 1203 communicate with each other through the communication bus 1204;
the memory 1203 stores a computer program, and the processor 1201 implements the steps of any of the above methods when the program is executed by the processor 1201.

The present application also provides a computer-readable storage medium, where a computer program executable by an electronic device is stored on the computer-readable storage medium, and the program is executed by the electronic device to implement the steps of any of the above methods when the program runs on the electronic device.

Although preferred embodiments of the present application have been described, those skilled in the art may make additional changes and modifications to these embodiments once they become aware of basic inventive concepts. Therefore, these appended claims are intended to be interpreted to include preferred embodiments and all changes and modifications falling within the scope of the present application.

It will be apparent to those skilled in the art that various modifications and variations can be made to the present application without departing from the spirit or scope of the present application. Thus, if these modifications and variations of the present application fall within the scope of the claims of the present application and their equivalents, the present application is also intended to include these modifications and variations.

## Claims

1. A health monitoring system, **characterized in that**, the system comprises a control device, a first health monitoring device, and a first drug infusion device;
wherein the control device is configured to connect to the first health monitoring device and the first drug infusion device respectively for mutual authentication, and activate the first health monitoring device and the first drug infusion device;
wherein the first health monitoring device is configured to broadcast a first data packet, wherein the first data packet contains first identification information of the first health monitoring device;
wherein the control device is configured to receive the first data packet, obtain the contained first identification information, and send the first identification information to the first drug infusion device;
wherein the first drug infusion device is configured to scan a connectable health monitoring device, match with the first health monitoring device based on the first identification information, and establish a connection to the first health monitoring device;
wherein the first health monitoring device is configured to obtain target health monitoring data of a first user and send the target health monitoring data to the first drug infusion device;
wherein the first drug infusion device is configured to send the target health monitoring data to the control device, receive a drug infusion index corresponding to the target health monitoring data from the control device, and infuse the drug to the first user based on the drug infusion index.

2. The system according to claim 1, wherein the first health monitoring device is configured to obtain target health monitoring data of a first user, encrypt the target health monitoring data with a first shared key, and send the encrypted target health monitoring data to the first drug infusion device;
wherein the first drug infusion device is configured to decrypt the target health monitoring data with the first shared key, encrypt the decrypted data with a second shared key, and send the data encrypted with the second shared key to the control device, so that the control device decrypts the data with the second shared key after receiving the data to obtain the target health monitoring data;
wherein the first drug infusion device receives a drug infusion index corresponding to the target health monitoring data encrypted with a third shared key from the control device, decrypts the data with the third shared key to obtain the drug infusion index, and infuses the drug to the first user based on the drug infusion index.

3. The system according to claim 1, wherein the system further comprises a server;
wherein the control device is configured to awaken the first drug infusion device through NFC;
wherein the first drug infusion device is configured to broadcast a second data packet, wherein the second data packet contains second identification information of the first drug infusion device;
wherein the control device is configured to receive the second data packet, obtain the contained second identification information, and send the second identification information to the server for verification;
wherein the server is configured to return first authentication information to the control device after the verification is passed;
wherein the control device is configured to connect to the first drug infusion device and perform mutual authentication based on the first authentication information, generate a fourth shared key after successful authentication, and activate the first drug infusion device;
wherein the first drug infusion device is configured to generate the fourth shared key and return an activation success notification.

4. The system according to claim 3, wherein the control device is configured to awaken the first health monitoring device through NFC;
wherein the first health monitoring device is configured to broadcast a third data packet, wherein the third data packet contains first identification information of the first health monitoring device;
wherein the control device is configured to receive the third data packet, obtain the contained first identification information, and send the first identification information to the server for verification;
wherein the server is configured to return second authentication information to the control device after the verification is passed;
wherein the control device is configured to connect to the first health monitoring device and perform mutual authentication based on the second authentication information, generate a fifth shared key after successful authentication, and activate the first health monitoring device;
wherein the first health monitoring device is configured to generate the fifth shared key and return an activation success notification.

5. The system according to claim 4, wherein the control device is configured to randomly generate third authentication information, encrypt the third authentication information with the fifth shared key, and send the encrypted third authentication information to the first health monitoring device;
wherein the first health monitoring device is configured to decrypt the encrypted information with the fifth shared key to obtain the third authentication information;
wherein the control device disconnects from the first health monitoring device;
wherein the control device encrypts the first identification information and the third authentication information with the fourth shared key and sends them to the first drug infusion device;
wherein the first drug infusion device is configured to decrypt the encrypted information with the fourth shared key to obtain the third authentication information and the first identification information;
wherein the first health monitoring device is configured to broadcast a fourth data packet, wherein the fourth data packet contains first identification information of the first health monitoring device;
wherein the first drug infusion device is configured to scan a connectable health monitoring device, match with the first health monitoring device based on the first identification information, and establish a connection to the first health monitoring device;
wherein the first drug infusion device is configured to perform mutual authentication with the first health monitoring device based on the third authentication information, generate a sixth shared key after successful authentication, and return first state information indicating the successful binding of the first health monitoring device to the control device.

6. The system according to claim 1, wherein the system further comprises a second health monitoring device;
wherein the first drug infusion device is further configured to disconnect from the first health monitoring device when identifying that the first health monitoring device expires and fails, and send second state information indicating the disconnection from the first health monitoring device and third state information indicating the expiration of the first health monitoring device to the control device;
wherein the control device is further configured to awaken the second health monitoring device through NFC after receiving the second state information and the third state information;
wherein the second health monitoring device is configured to broadcast a fifth data packet, wherein the fifth data packet contains third identification information of the second health monitoring device;
wherein the control device is configured to receive the fifth data packet, obtain the contained third identification information, connect to the second health monitoring device for mutual authentication, activate the second health monitoring device, and send the third identification information to the first drug infusion device;
wherein the control device is configured to randomly generate fourth authentication information, encrypt the fourth authentication information, and send the encrypted fourth authentication information to the second health monitoring device and the first drug infusion device;
wherein the first drug infusion device is configured to scan a connectable health monitoring device, match with the second health monitoring device based on the third identification information, and connect to the second health monitoring device for mutual authentication based on the fourth authentication information.

7. The system according to claim 6, wherein the system further comprises a second drug infusion device;
wherein the control device is further configured to connect to a recently activated third health monitoring device when identifying that the first drug infusion device expires and fails and the second drug infusion device is not detected, to obtain health monitoring data collected by the third health monitoring device;
wherein the control device is further configured to awaken the second drug infusion device through NFC, perform mutual authentication and activate the second drug infusion device when detecting the second drug infusion device, and send fourth identification information of the third health monitoring device to the second drug infusion device;
wherein the control device is configured to randomly generate fifth authentication information, encrypt the fifth authentication information, and send the encrypted fifth authentication information to the third health monitoring device and the second drug infusion device;
wherein the second drug infusion device is configured to scan a connectable health monitoring device, match with the third health monitoring device based on the fourth identification information, and connect to the third health monitoring device for mutual authentication based on the fifth authentication information.

8. The system according to any one of claims 1 to 7, wherein the control device is further configured to disconnect from a third drug infusion device when detecting itself away from the third drug infusion device and a fourth health monitoring device in a connected state;
wherein the third drug infusion device is configured to receive health monitoring data from the fourth health monitoring device after detecting the disconnection from the control device, determine a drug infusion index corresponding to the health monitoring data, and infuse the drug to the first user based on the drug infusion index.

9. The system according to claim 8, wherein the third drug infusion device is further configured to broadcast a sixth data packet after the disconnection from the control device, and the sixth data packet contains fifth identification information of the third drug infusion device;
wherein the control device is further configured to scan a connectable drug infusion device, match with the third drug infusion device based on the fifth identification information, and establish a connection to the third drug infusion device;
wherein the third drug infusion device is configured to receive health monitoring data from the fourth health monitoring device after detecting that a connection to the control device is established and the mutual authentication is successful, send the health monitoring data to the control device, receive a drug infusion index corresponding to the health monitoring data from the control device, and infuse the drug to the first user based on the drug infusion index.

10. A health monitoring method, **characterized in that**, the method is applied to a first drug infusion device and comprises:
scanning, by the first drug infusion device, a connectable health monitoring device, matching with a first health monitoring device based on first identification information of the first health monitoring device, and establishing a connection to the first health monitoring device, wherein a control device receives a first data packet broadcast by the first health monitoring device, obtains the contained first identification information, and sends the first identification information to the first drug infusion device;
receiving target health monitoring data of a first user from the first health monitoring device, sending the target health monitoring data to the control device, receiving a drug infusion index corresponding to the target health monitoring data from the control device, and infusing the drug to the first user based on the drug infusion index.

11. A health monitoring method, **characterized in that**, the method is applied to a first health monitoring device and comprises:
broadcasting, by the first health monitoring device, a first data packet, wherein the first data packet contains first identification information of the first health monitoring device; a control device receives the first data packet, obtains the contained first identification information, and sends the first identification information to a first drug infusion device; the first drug infusion device scans a connectable health monitoring device, matches with the first health monitoring device based on the first identification information, and establishes a connection to the first health monitoring device;
obtaining target health monitoring data of a first user and sending the target health monitoring data to the first drug infusion device; the first drug infusion device sends the target health monitoring data to the control device, receives a drug infusion index corresponding to the target health monitoring data from the control device, and infuses the drug to the first user based on the drug infusion index.

12. A health monitoring method, **characterized in that**, the method is applied to a control device and comprises:
connecting, by the control device, to the first health monitoring device and the first drug infusion device respectively for mutual authentication, and activating the first health monitoring device and the first drug infusion device;
receiving a first data packet broadcast by the first health monitoring device, obtaining the contained first identification information, and sending the first identification information to the first drug infusion device; the first drug infusion device scans a connectable health monitoring device, matches with the first health monitoring device based on the first identification information, and establishes a connection to the first health monitoring device;
receiving target health monitoring data of a first user from the first drug infusion device, wherein the target health monitoring data is sent from the first health monitoring device to the first drug infusion device;
determining a drug infusion index corresponding to the target health monitoring data, and sending the drug infusion index to the first drug infusion device, so that the first drug infusion device infuses the drug to the first user based on the drug infusion index.

13. A drug infusion device, **characterized in that**, it comprises a processor, a communication interface, a memory, and a communication bus, and wherein the processor, the communication interface and the memory communicate with each other through the communication bus;
wherein the memory is configured to store the computer program;
wherein the processor is configured to implement the steps of the method according to claim 10 when executing the program stored in the memory.

14. A health monitoring device, **characterized in that**, it comprises a processor, a communication interface, a memory, and a communication bus, and wherein the processor, the communication interface and the memory communicate with each other through the communication bus;
wherein the memory is configured to store the computer program;
wherein the processor is configured to implement the steps of the method according to claim 11 when executing the program stored in the memory.

15. A control device, **characterized in that**, it comprises a processor, a communication interface, a memory, and a communication bus, and wherein the processor, the communication interface and the memory communicate with each other through the communication bus;
wherein the memory is configured to store the computer program;
wherein the processor is configured to implement the steps of the method according to claim 12 when executing the program stored in the memory.
